# EUROPÄISCHE PATENTSCHRIFT

(11) **EP 0 298 100 B1**
(45) Veröffentlichungstag und Bekanntmachung des Hinweises auf die Patenterteilung: **17.03.1993**
(21) Anmeldenummer: 88900768.8
(22) Anmeldetag: 14.01.1988
(51) Int. Cl.: A61B 17/56

(54) **OSTEOSYNTHESEHILFSMITTEL**
BONE SYNTHESIS AID
DISPOSITIF D'AIDE A L'OSTEOSYNTHESE

(30) Priorität: 21.01.1987 DE 3701533
(43) Veröffentlichungstag der Anmeldung: 11.01.1989
(73) Patentinhaber: ORTHOFIX S.r.l., 37012 Bussolengo (VR) (IT)
(72) Erfinder: PENNIG, Dietmar, Dr., W-4400 Münster/W. (DE)
(74) Vertreter: Habbel, Hans-Georg, Dipl.-Ing.
(86) Internationale Anmeldenummer: DE8800017
(87) Internationale Veröffentlichungsnummer: WO8805287

(56) Entgegenhaltungen:
- EP-A- 0 011 258
- CH-A- 169 173
- CH-A- 203 544
- US-A- 1 789 060

## Beschreibung

Die Erfindung bezieht sich auf ein Osteosynthesehilfsmittel gemäß dem Oberbegriff des Hauptanspruches.

Osteosynthesehilfsmittel, insbesondere metallische Hilfsmittel , dienen der Fixation einer Fraktur. Bei gewinkelten, gewölbten oder gekrümmten Knochensegmenten, beispielsweise bei der Kieferchirurgie, ist die Festlegung der Frakturenden in der genau gewünschten und erforderlichen Stellung eine sehr schwierig durchzuführende Arbeit, insbesondere dann, wenn - wie dies heute üblich ist - die Hilfsmittel vollkommen gedeckt, also intern, angebracht werden sollen.

Aus der US-A-17 89 060 ist ein gattungsbildendes Osteosynthesehilfsmittel bekannt. Bei diesem Hilfsmittel erfolgt die Fixation der Fraktur durch ein außerhalb des menschlichen Körpers angeordnetes Hilfsmittel, und dieses Hilfsmittel besteht im wesentlichen aus zwei Halteplatten, die die Fixationsmittel in Längsachse der Halteplatte und in ihrer Höhenlage gegenüber der Halteplatte einstellbar tragen. Eine Schwenkbewegung jeweils in einer Ebene, zu der die Längsachse der zugeordneten Halteplatte parallel verläuft und eine Festlegung in der verschwenkten Stellung der Fixationsmittel an den Halteplatten ist nicht möglich. Die beiden Halteplatten schließen über ein kompliziertes Gelenk aneinander an, das einerseits aus zwei gegeneinander verstellbaren Kugelhälften besteht, die miteinander arretierbar sind, andererseits aus je einer Langlochführung an jeder Halteplatte, wobei die Langlöcher der beiden Halteplatten senkrecht zueinander stehen, so daß die eine Halteplatte in ihrer Höhe und die andere Halteplatte in ihrer Querstellung eingeregelt werden kann. Mit dieser Vorrichtung ist nur eine diskontinuierliche Möglichkeit für das Einrichten des Knochens gegeben, und bei Frakturen kompliziert gewölbter Knochen, wie beispielsweise Kieferknochen oder Rückenwirbel , ist ein Einrichten der Fraktur aufgrund der begrenzten Freiheitsgrade des Osteosynthesehilfsmittels nur begrenzt möglich.

Aus der EP-A-11 258 ist eine Vorrichtung zur äußeren Festlegung der Teile eines gebrochenen Knochens bekanntgeworden, bei der nicht die Möglichkeit besteht, die Fixationsmittel an jedem Halteelement in Längsachse des Halteelementes verstellbar und arretierbar anzuordnen. Die Fixationsmittel sind vielmehr ortsfest festgelegt und können nicht unabhängig von der Stellung der zugeordneten Halteelemente in ihrer Ausrichtung eingestellt werden. Die Halteelemente greifen unter Zwischenschaltung von Kugelgelenken an einem längenverstellbaren Distanzstück - einem Teleskopstück - an, wobei die Schwenkbewegung zwischen jedem Halteelement und dem Teleskopstück auf 18^{o} in jeder Richtung beschränkt ist.

Der Erfindung liegt die Aufgabe zugrunde, ein Osteosynthesehilfsmittel zur Fixation von Frakturen zu schaffen, das einen großen Verschwenkwinkel der beiden Halteplatten gegeneinander ermöglicht und bei welchem das Einrichten des Knochens kontinuierlich erfolgen kann, so daß dadurch die Arbeit des Operateurs erleichtert wird.

Diese der Erfindung zugrundeliegende Aufgabe wird durch die Lehre des Hauptanspruches gelöst.

Vorteilhafte und sinnvolle Ausgestaltungen sind in den Unteransprüchen erläutert.

Mit anderen Worten ausgedrückt, wird ein Osteosynthesehilfsmittel vorgeschlagen, bei welchem über das Doppelkugelgelenk und das zwischen den beiden Kugelköpfen angeordnete Distanzstück ein großer Verschwenkwinkel der beiden Halteplatten gegeneinander möglich wird und bei welchem die Verstellung jeder einzelnen Halteplatte für sich in allen Richtungen möglich wird.

Durch die zusätzlich gemäß der Erfindung vorgesehene schwenkbare und höhenverstellbare Festlegung der Fixationsmittel an den Halteplatten werden zusätzliche Freiheitsgrade für die Gesamtvorrichtung geschaffen, die es ermöglichen, auch den kompliziertesten Knochenstellungen und Einrichtarbeiten gerecht zu werden.

Mit dem erfindungsgemäßen Osteosynthesehilfsmittel ist es daher möglich, zuerst die Fixationsmittel in den miteinander zu verbindenen Knochensegmenten anzubringen, dann die Halteplatten anzuschließen, dann die Halteplatten in ihrem gewünschten Winkel, beispielsweise unter einem Röntgenschirm einzustellen und die beiden Knochenteile aufeinander einzurichten, um dann das gesamte Osteosynthesehilfsmittel hinsichtlich seiner Verstellbarkeit absolut festzulegen. Mit der erfindungsgemäßen Anordnung wird den üblicherweise auftretenden drei möglichen Fehlstellungen Rechnung getragen, nämlich einerseits der Drehung der beiden getrennten Knochenteile und der beiden in zwei verschiedenen Ebenen gerichteten Verschiebungen der beiden Knochenteile.

Ein Ausführungsbeispiel der Erfindung wird nachfolgend anhand der Zeichnung erläutert. Die Zeichnung zeigt dabei in
- Fig. 1: schaubildlich das Osteosynthesehilfsmittel in seiner Gesamtansicht und in
- Fig. 2: in einer auseinandergezogenen Darstellungsweise die einzelnen Bauteile der in Fig. 1 dargestellten Einrichtung zur Verdeutlichung der Funktion der einzelnen Elemente.

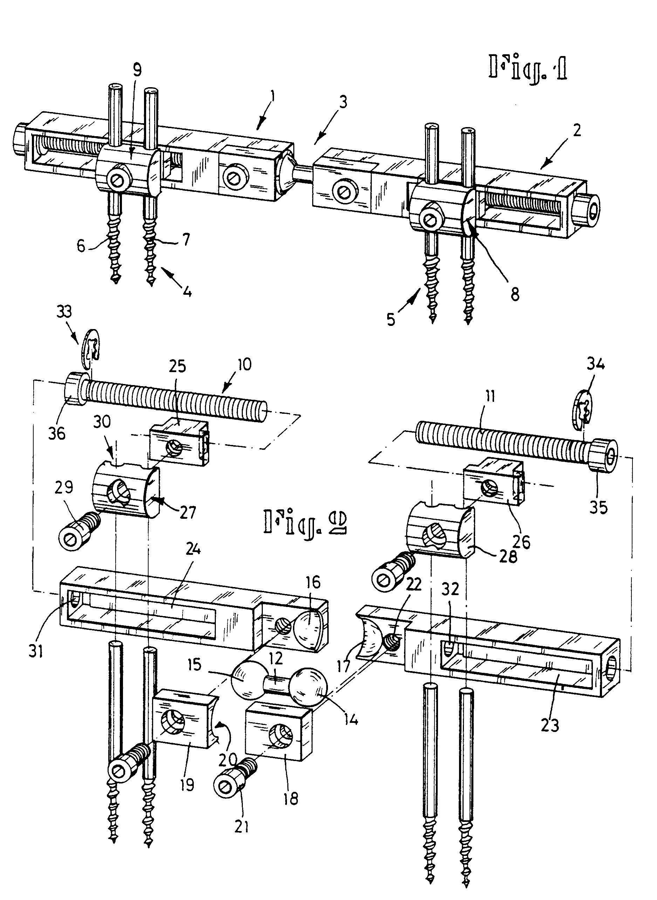

In Fig. 1 sind mit 1 und 2 zwei Halteplatten bezeichnet, die über ein Doppelkugelgelenk 3 miteinander verbunden sind. Die Halteplatten tragen Fixationsmittel 4 und 5, wobei die Fixationsmittel bei dem dargestellten Ausführungsbeispiel jeweils aus zwei Schrauben 6 und 7 bestehen, die an den Halteplatten festlegbar sind.

Die Fixationsmittel sind an den Halteplatten über Befestigungsböckchen 8 und 9 festgelegt, wobei diese Befestigungsböckchen an den Halteplatten 1 und 2 in deren Längsachse verstellbar gelagert sind. Die Festlegung der Befestigungsböckchen 8 und 9 und ihre Verstellung erfolgt je über eine Schraubspindel 10 und 11.

Das Doppelkugelgelenk 3 besteht aus zwei über ein starres Distanzstück 12 miteinander verbundenen Kugelköpfen 14 und 15, die im Endbereich jeder Halteplatte 1 bzw. 2 in viertelkugelförmigen Kugelpfannen 16 und 17 liegen. Die Festlegung der Kugelköpfe erfolgt über Kugelpfannenplatten 18 und 19, die ebenfalls mit viertelkugelpfannenförmigen Ausnehmungen ausgerüstet sind, wobei in Fig. 2 die viertelpfannenförmige Ausnehmung 20 der Kugelpfannenplatte 19 erkennbar ist. Die Befestigung der Kugelpfannenplatten 18 und 19 erfolgt über Schrauben 21, die sich in entsprechende Bohrungen 22 in den Halteplatten einschrauben. Durch mehr oder weniger starkes Festziehen der Schrauben 21 ist es möglich, daß das Doppelkugelgelenk 3 allseits beweglich ist oder verstarrt wird.

In den Halteplatten 1 und 2 sind Kulissenführungen 23 und 24 vorgesehen, in denen sich je ein Kulissenstein 25 bzw. 26 führt, auf den jeweils ein Klemmmittel 27 bzw. 28 beispielsweise über die Schraube 29 aufgeschraubt werden kann. Jedes Klemmittel 27 oder 28 weist einen der Form der Nägel 6 bzw. 7 angepaßten Aufnahmeraum 30 auf, wobei bei dem dargestellten Ausführungsbeispiel jedes Klemmittel 27 zwei solcher Aufnahmeräume aufweist. Durch entsprechendes Festschrauben der Schraube 29 ist also auch die Stellung der Nägel 6 und 7 arretierbar. Bei Lockern der Schraube 29 ist ein Verdrehen des Klemmittels 27 aus der in Fig. 1 dargestellten Stellung möglich, so daß dadurch auch die Richtung der Schrauben 6 und 7 eingeregelt werden kann.

Die Kulissensteine 25 und 26 greifen in die Kulissenführungen 23 und 24 ein und weisen - was aus der Zeichnung nur schwer zu erkennen ist - eine mit Innengewinde ausgerüstete Bohrung auf, die mit dem Außengewinde der Schraubspindel 10 bzw. 11 kämmt. Jede Schraubspindel 10 bzw. 11 wird in der Kulissenführung 23 bzw. 24 dadurch festgelegt, daß sich der Schaft der Schraubspindel 10 bzw. 11 in je einer Öffnung 31 bzw. 32 führt und ein Federschnappring 33 bzw. 34 auf den Schaft aufgesetzt ist. Das Gegenlager für den Federschnappring 33 bzw. 34 bildet der verdickte Kopf 35 bzw. 36 der Schraubspindel 10.

## Patentansprüche

1. Osteosynthesehilfsmittel für die Fixation gewölbter oder gekrümmter Knochen mit zwei jeweils Fixationsmittel (4, 5) tragenden Halteplatten (1, 2), die über ein arretierbares Gelenk miteinander verbunden sind, wobei die Fixationsmittel (4, 5), wie Schrauben (6, 7) oder Nägel, an jeder Halteplatte (1, 2) in deren Längsachse und in der Höhe arretierbar verstellbar sind, dadurch gekennzeichnet, daß das Gelenk als Doppelkugelgelenk (3) ausgebildet ist, dessen zwei Kugelköpfe (14, 15) über ein starres Distanzstück (12) miteinander verbunden sind und die Fixationsmittel (4, 5) jeweils in einer Ebene zu der die Längsachse der zugeordneten Halteplatte (1, 2) parallel verläuft schwenkbar und in der verschwenkten Stellung festlegbar an jeder Halteplatte angeordnet sind.

2. Osteosynthesehilfsmittel nach Anspruch 1, dadurch gekennzeichnet, daß in den Halteplatten (1, 2) endseitig Kugelpfannen (16, 17) ausgenommen sind, die der Aufnahme der Kugelköpfe (14, 15) dienen, wobei die Kugelpfannen (16, 17) im wesentlichen viertelkugelförmig gestaltet sind und die Festlegung der Kugelköpfe (14, 15) in den Kugelpfannen über je eine Kugelpfannenplatte (18, 19) erfolgt, die je ebenfalls eine im wesentlichen viertelkugelpfannenförmige Ausnehmung (20) aufweist, wobei die Kugelpfannenplatten (18, 19) an den Halteplatten (1, 2) eine Arretierung der Kugelköpfe bewirkend festlegbar sind.

3. Osteosynthesehilfsmittel nach Anspruch 1 oder 2, dadurch gekennzeichnet, daß an jeder Halteplatte (1, 2) ein Befestigungsbock (8, 9) in Längsachse der Halteplatte (1, 2) verstellbar angeordnet ist, der mit Klemmitteln (27, 28) zur schwenkbaren und höhenverstellbaren Festlegung der Fixationsmittel (4, 5) ausgerüstet ist.

4. Osteosynthesehilfsmittel nach Anspruch 3, dadurch gekennzeichnet, daß der Befestigungsbock (8, 9) durch einen Kulissenstein (25, 26) gebildet wird, der in einer Kulissenführung (23, 24) der Halteplatte (1, 2) verschiebbar gelagert ist und über eine in der Halteplatte (1, 2) frei drehbar gelagerte Schraubspindel (10, 11) verstellbar ist, die mit einer mit Innengewinde ausgerüsteten Bohrung des Kulissensteines (25, 26) kämmt.

5. Osteosynthesehilfsmittel nach Anspruch 4, dadurch gekennzeichnet, daß die nach außen gerichtete Oberseite des Kulissensteines (25, 26) eben ausgebildet ist und das an dem Kulissenstein (25, 26) festlegbare Klemmittel (27, 28) an seiner zum Kulissenstein hin gerichteten Seite dem Fixationsmittel (4, 5) angepaßte Aufnahmeräume (30) aufweist.

6. Osteosynthesehilfsmittel nach Anspruch 4 oder 5, dadurch gekennzeichnet, daß die Festlegung der Schraubspindeln (10, 11) gegen eine in Längsachse der Halteplatten gerichtete Bewegung innerhalb der Kulissenführungen (23, 24) durch einen Federschnappring (33, 34) erfolgt.

## Claims

1. A bone synthesis aid for fixing curved or bent bones comprising two support plates (1, 2) each carrying fixing means (4, 5), which support plates (1, 2) are connected together by means of an arrestable joint, it being possible for the fixing means (4, 5), such as screws (6, 7) or nails, to be arrestably adjusted longitudinally and vertically at each support plate (1, 2), characterized in that the joint is constructed as a double ball-and-socket joint (3), whose two ball heads (14, 15) are connected together via a rigid spacer member (12) and in that the fixing means (4, 5) are each arranged so as to be swivellable and securable in the swivelled position on each support plate in a plane to which the longitudinal axis of the associated support plate (1, 2) runs parallel.

2. A bone synthesis aid according to claim 1, characterized in that ball sockets (16, 17) are recessed in the support plates (1, 2) at the ends thereof, said ball sockets (16, 17) serving to accommodate the ball heads (14, 15) and being substantially in the form of quadrants and fixing of each of the ball heads (14, 15) in the ball sockets being effected by a ball socket plate (18, 19), each of which likewise comprises a substantially quadrant socket-form recess (20), it being possible to secure the ball socket plates (18, 19) to the support plates (1, 2) to effect arresting of the ball heads.

3. A bone synthesis aid according to claim 1 or claim 2, characterized in that a fastening bracket (8, 9) is arranged adjustably on each support plate (1, 2) in the longitudinal axis of the support plate (1, 2), which bracket (8, 9) is equipped with clamping means (27, 28) for swivellable and vertically adjustable securing of the fixing means (4, 5).

4. A bone synthesis aid according to claim 3, characterized in that the fastening bracket (8, 9) is formed by a sliding block (25, 26), which is accommodated displaceably in a sliding guide (23, 24) in the support plate (1, 2) and can be adjusted by means of a screw spindle (10, 11) accommodated freely rotatably in the support plate (1, 2), which screw spindle (10, 11) meshes with a bore in the sliding block (25, 26) provided with an internal thread.

5. A bone synthesis aid according to claim 4, characterized in that the outwardly directed top of the sliding block (25, 26) is of planar construction and in that the clamping means (27, 28) securable to the sliding block (25, 26) comprises accommodation spaces (30) conformed to the fixing means (4, 5) on its side facing the sliding block.

6. A bone synthesis aid according to claim 4 or claim 5, characterized in that securing of the screw spindles (10, 11) against movement inside the sliding guides (23, 24) in the direction of the longitudinal axis of the support plates is effected by a spring-loaded snap ring (33, 34).

## Revendications

1. Dispositif auxiliaire d'ostéosynthèse pour la fixation d'os bombés ou courbes, comprenant deux plaques de monture (1, 2) portant chacune des moyens de fixation (4, 5), et qui sont reliées l'une à l'autre par une articulation blocable, les moyens de fixation (4, 5), tels que des vis (6, 7) ou clous, étant réglables sur chaque plaque de monture (1, 2) selon son axe longitudinal et en hauteur et pouvant être bloquées en position, caractérisé en ce que l'articulation est constituée par une articulation à rotule double (3) dont les deux têtes de rotules (14, 15) sont reliées l'une à l'autre par une entretoise rigide (12) et les moyens de fixation (4, 5) sont disposés de façon à pouvoir s'incliner dans un plan auquel l'axe longitudinal de la plaque de monture correspondante (1, 2) est parallèle et à pouvoir être bloqués sur chaque plaque de monture dans la position inclinée.

2. Dispositif auxiliaire d'ostéosynthèse selon la revendication 1, caractérisé en ce que, dans les plaques de monture (1, 2) sont évidées, en bout, des cuvettes (16, 17) de rotules qui servent à recevoir les têtes (14, 15) de rotules, les cuvettes (16, 17) de rotules présentant sensiblement la forme de quarts de sphère et l'immobilisation des têtes (14, 15) de rotules dans les cuvettes de rotules étant réalisée, pour chacune, par l'intermédiaire d'une plaque (18, 19) de cuvette de rotule, qui présente elle aussi un évidement (20) formant une cuvette sensiblement en quart de sphère, les plaques (18, 19) de cuvettes de rotules pouvant être immobilisées sur les plaques de monture (1, 2) en déterminant ainsi un blocage des têtes de rotules.

3. Dispositif auxiliaire d'ostéosynthèse selon la revendication 1 ou 2, caractérisé en ce qu'à chaque plaque de monture (1, 2), est associé un bloc de fixation (8, 9) réglable selon l'axe longitudinal de la plaque de monture (1, 2), et qui est équipé de moyens de serrage (27, 28) pour l'immobilisation des moyens de fixation (4, 5) avec possibilité d'inclinaison et de réglage en hauteur.

4. Dispositif auxiliaire d'ostéosynthèse selon la revendication 3, caractérisé en ce que le bloc de fixation (8, 9) est formé d'un coulisseau (25, 26) qui est monté coulissant dans une coulisse (23, 24) de la plaque de monture (1, 2) et peut être réglé au moyen d'une vis (10, 11) qui est montée librement rotative dans la plaque de monture (1, 2), et qui est en prise avec un perçage du coulisseau (25, 26) muni d'un filetage intérieur.

5. Dispositif auxiliaire d'ostéosynthèse selon la revendication 4, caractérisé en ce que la surface du coulisseau (25, 26) qui est dirigée vers l'extérieur est plane et le moyen de serrage (27, 28) qui peut être immobilisé sur le coulisseau (25, 26) présente sur sa face dirigée vers le coulisseau, des chambres de réception (30) adaptées au moyen de fixation (4, 5).

6. Dispositif auxiliaire d'ostéosynthèse selon la revendication 4 ou 5, caractérisé en ce que l'immobilisation des vis (10, 11) à l'encontre d'un déplacement à l'intérieur des coulisses (23, 24) selon l'axe longitudinal des plaques de monture est assurée par une bague d'arrêt élastique (33, 34).
